# EUROPEAN PATENT SPECIFICATION

(11) **EP 2 884 956 B1**
(45) Date of publication and mention of the grant of the patent: **21.03.2018**
(21) Application number: 13739437.5
(22) Date of filing: 22.07.2013
(51) Int. Cl.: A61K 8/00, A61K 31/728, A61K 31/737, A61P 3/10

(54) **COMPOSITIONS COMPRISING HYALURONIC ACID AND DERMATAN SULPHATE FOR THE TREATMENT OF BEING OVERWEIGHT AND OBESITY**
ZUBEREITUNGEN MIT HYALURONSÄURE UND DERMATANSULPHAT ZUR BEHANDLUNG VON ÜBERGEWICHT UND FETTLEIBIGKEIT
COMPOSITIONS COMPRENANT DE L'ACIDE HYALURONIQUE ET DU DERMATANE SULFATE POUR LE TRAITEMENT DU FAIT D'ÊTRE EN SURPOIDS ET DE L'OBÉSITÉ

(30) Priority: 25.07.2012 ES 201231198
(43) Date of publication of application: 24.06.2015
(73) Proprietor: BIOIBERICA, S.A., 08029 Barcelona (ES)
(72) Inventor: ESCAICH FERRER, Josep, E-08021 Barcelona (ES); BONET PIÑA, Mª Luisa, E-07010 Palma De Mallorca (ES); MARTINEZ PUIG, Daniel, E-08500 Vic (ES); GRANADOS BORBOLLA, Nuria, E-07005 Palma De Mallorca (ES); REYNES MIRALLES, Barbara, E-07360 Lloseta (ES); CHETRIT RUSSI, Carles, E-17820 Banyoles (ES); PALOU OLIVER, Andreu, E-07015 Palma De Mallorca (ES)
(74) Representative: Sugrañes Patentes y Marcas
(86) International application number: PCT/EP2013/065384
(87) International publication number: WO 2014/016233

(56) References cited:
- US-A1- 2007 196 438
- DATABASE WPI Week 201023 Thomson Scientific, London, GB; AN 2010-D03903 XP002711300, & JP 2010 059084 A (QP CORP) 18 March 2010 (2010-03-18)

## Description

### Technical Field of the Invention

The present invention relates to compositions for use in the treatment, prevention or reduction of overweight or obesity, as well as of metabolic diseases, conditions or alterations related to overweight or obesity, such as insulin resistance, type 2 diabetes, fatty liver or dyslipidemia. The compositions can be in the form of pharmaceutical compositions, foods, functional foods, medical foods or food supplements.

### Background of the invention

Obesity, which is defined as excess body weight in the form of fat, is an important risk factor for serious clinical disorders, including type 2 diabetes and cardiovascular disease, and it is a problem of increasing incidence and difficult treatment that leads to a considerable social-health expense (see http://www.iaso.org/iotf/obesity/). It is known that many people with overweight or obesity develop insulin resistance and hyperinsulinemia, glucose intolerance, dyslipidemia and hypertension, which are all characteristics that together define the so-called metabolic syndrome (P.L. Huang, Dis. Model. Mech. 2(5-6), 231-237 (2009)). It is also known that most obese people have a state of hyperleptinemia and leptin resistance. The results of various research projects suggest that leptin is an important factor linking obesity, metabolic syndrome, hypertension and cardiovascular alterations (S.B. Patel et al., Curr. Hypertens. Rep. 10, 131-137 (2008)). The presence of resistance to the action of leptin in obesity was acknowledged some time ago (P.S. Widdowson et al., Diabetes 46, 1782-1785 (1997)). Since then, the results of a number of studies have demonstrated that maintaining high leptin levels reduces the expression of leptin receptors and deteriorates the leptin signaling pathway in the central nervous system, leading to leptin resistance which in turn confers greater susceptibility to obesity and its medical complications (Y. Zhang & P.J. Scarpace, Physiol. Behav. 88, 249-256 (2006)). Essentially, if circulating leptin levels remain high, they contribute to leptin resistance and promote overweight and obesity, reinforcing the vicious circle of multiple metabolic alterations occurring with obesity and defining metabolic syndrome, especially in the conditions of developed societies in which there is an overabundance of energy-rich foods (P.J. Scarpace & Y. Zhang, Front Biosci. 12, 3531-3544 (2007)).

One of the most common metabolic complications in obesity is insulin resistance (A. Astrup & N. Finer, Obes. Rev. 1, 57-59 (2000)). The connection between both conditions has been widely studied and related to the fact that the adipose tissue of obese individuals releases increased or abnormal amounts of non-esterified fatty acids, hormones (adipokines) and proinflammatory cytokines which favor the loss of insulin sensitivity in different tissues (S.E. Kahn et al., Nature 444, 840-846 (2006)). Resistin, in particular, is a protein secreted by adipocytes and/or macrophages infiltrated in obese adipose tissue which acts as a proinflammatory and insulin resistance factor, the circulating levels of which are high in obese individuals (D.R. Schwartz & M.A. Lazar, Trends Endocrinol. Metab. 22, 259-265 (2011)). The reduction of resistin is correlated with increases in insulin sensitivity (C.M. Steppan et al., Nature 409, 307-312 (2001); F. Felipe et al., Diabetes 53, 882-889 (2004)).

Insulin resistance is clinically relevant because it is closely associated with type 2 diabetes and other problems such as hypertension, dyslipidemia, and blood coagulation defects and fibrinolysis, all of which are in turn related with cardiovascular disease (G. Boden, Curr. Opin. Endocrinol. Diabetes Obes. 18, 139-143 (2011)). It is generally accepted that the primary defect in the development of type 2 diabetes is the deterioration of insulin sensitivity followed by compensatory hyperinsulinemia, and hyperglycemia when the compensation mechanisms fail. Therefore, the improvement of glucose-insulin homeostasis is a health objective of specific interest itself.

Weight reduction strategies based only on calorie restriction and the increase of physical activity are difficult to follow and have proven ineffective against the actual pandemic of obesity. In parallel, evidence is accumulating that specific nutrients and other food ingredients affect biochemical processes that impact the energy balance and fat accumulation in a manner such that they can favor a leaner phenotype (K. H. Kim & Y. Park, Annu. Rev. Food Sci. Technol. 2, 237-257 (2011); C. Pico et al., Rev. Esp. Obes. 4, 156-174 (2006); E. M. Kovacs & D. J. Mela, Obes. Rev. 7, 59-78 (2006)). This evidence is the basis for the development of food supplements or functional foods for controlling overweight and obesity.

The following main approaches to functional foods for controlling obesity have been defined: (i) reduction of energy intake, by modulating hunger and/or satiety, or limiting the bioavailability of nutrients; (ii) reduction of caloric density in light foods; (iii) stimulation of energy expenditure through activation of adaptive thermogenesis; and (iv) re-routing nutrients towards tissues and metabolic pathways that consume them, hence avoiding fat deposition (C. Pico et al., Rev. Esp. Obes. 4, 156-174 (2006)). The use of nutrients or combinations of nutrients able to simultaneously affect several of these processes is considered to be of special interest (C Pico et al., Rev. Esp. Obes. 4, 156-174 (2006)).

Adipogenesis inhibition is another possible anti-obesity approach (K. H. Kim & Y. Park, Annu. Rev. Food Sci. Technol. 2, 237-257 (2011)). Adipogenesis is the process of differentiating adipocytes from precursor cells and has been widely studied, primarily in cell models (B. Feve, Best Pract. Res. Clin. Endocrinol. Metab. 19, 483-499B (2005)). In humans, a considerable percentage of adipocytes, around 10%, is renewed annually throughout the entire adult life by means of coordinating *de novo* adipogenesis and the death of pre-existing adipocytes (K. L. Spalding et al., Nature 453, 783-787 (2008)). Many obese individuals have an excess number of adipocytes (hyperplasia) in their fat depots, and these individuals are especially resistant to long-term weight loss and prone to the well-known yo-yo effect. Pharmacological or nutritional control of adipogenesis emerges in this context as a new therapeutic target in controlling obesity, as a coadjuvant in conventional energy balance control strategies: adipogenesis inhibition can aid in normalizing (reducing) the number of adipocytes, and thereby in maintaining the weight after weight loss (P. Arner & K. L. Spalding, Biochem. Biophys. Res. Commun. 396, 101-104 (2010)).

Glycosaminoglycans (GAGs) are high molecular weight polymer biomolecules formed by a repeating disaccharide unit. They are fundamentally found in living organisms where they carry out different physiological functions.

Hyaluronic acid is a non-sulphated glycosaminoglycan with a polymer structure characterised by a repeating disaccharide, made up of the monosaccharides *N*-acetyl-D-glucosamine and D-glucuronic acid. It is one of the main components of cartilage, of the synovial membrane and of synovial fluid. Its use in the treatment of joint dysfunctions such as osteoarthritis, generally by intra-articular route, is particularly important. Its use in ophthalmology, in wound healing and in cosmetics has also been described.

Dermatan sulphate is a sulphated glycosaminoglycan with a polymer structure made up primarily of disaccharides of *N*-acetyl-D-galactosamine sulphated in position 4 and L-iduronic acid, often sulphated in position 2. It has been described that it participates in wound repair and in blood clotting regulation. This substance is largely used in cosmetics.

Collagen hydrolysate is made up of a mixture of low molecular weight peptides and amino acids. It is obtained by controlled enzyme hydrolysis of the collagen protein contained in the skin and in other connective tissues. It is primarily used in cosmetics.

Patent application US 2005084518 describes a health food containing hyaluronic acid and dermatan sulphate. Said food is useful for producing beautiful skin.

Patent US 7,763,594 discloses the use of a composition containing hyaluronic acid and dermatan sulphate for treating osteoarthritis.

In view of the foregoing, providing compositions for the treatment, prevention or control of overweight and/or obesity, as well as of some of their metabolic complications, is a topic of enormous interest in both the therapeutic and nutritional fields.

### Disclosure of the Invention

The present inventors have surprisingly found that the compositions of the present invention defined below exert an anti-adipogenic synergistic effect, even in the presence of a pro-adipogenic hormone cocktail; inhibit the expression of resistin (an adipokine related to insulin resistance); reduce weight gain and energy intake in mice fed *ad libitum*; reduce insulinemia and triglyceridemia in mice fed *ad libitum*; favor weight and fat loss in obese mice; reduce leptinemia and insulinemia in obese mice; and regulate leptinemia, cholesterolemia and triglyceridemia in obese humans. Therefore, the compositions of the present invention can be used in the treatment or prevention of obesity, insulin resistance, type 2 diabetes, fatty liver or dyslipidemia, and can be in the form of a pharmaceutical composition or a medical food. The compositions can also be used as a food, functional food or food supplement for reducing overweight, reducing food intake, inducing the feeling of satiety, reducing appetite, reducing body weight, preventing weight gain, reducing body fat or reducing fat formation in a mammal.

All the diseases, conditions or alterations mentioned in the present invention are interrelated, because together they define the so-called metabolic syndrome, sharing common pathophysiological mediators and mechanisms. In order to treat or palliate them, they all share the common characteristic of needing to act on adipogenesis and/or on the levels of leptin, a hormone secreted by the adipocytes.

Therefore, according to a first aspect, the present invention relates to a composition comprising hyaluronic acid and dermatan sulphate for use in the treatment or prevention of obesity, insulin resistance, type 2 diabetes, fatty liver or dyslipidemia in a mammal.

In a preferred embodiment of the composition for use, insulin resistance is associated with overweight or with obesity, type 2 diabetes is associated with overweight or with obesity, fatty liver is associated with overweight or with obesity, or dyslipidemia is associated with overweight or with obesity.

The dyslipidemia is preferably hypercholesterolemia and/or hypertriglyceridemia.

In another preferred embodiment of the composition for use, the treatment or prevention of dyslipidemia results in reducing blood cholesterol, maintaining normal blood cholesterol levels, reducing blood triglycerides or maintaining normal blood triglyceride levels.

According to another preferred embodiment of the composition for use, the treatment or prevention of insulin resistance or type 2 diabetes results in increasing insulin sensitivity.

In another preferred embodiment of the composition for use, the composition is in the form of a pharmaceutical composition or a medical food.

According to another preferred embodiment of the composition for use, the mammal is obese or has overweight.

In further embodiments of the composition for use, the mammal has a metabolic syndrome or has hyperleptinemia, respectively.

In another also preferred embodiment, the mammal is a human.

In another preferred embodiment of the composition for use, hyaluronic acid and dermatan sulphate are present at a hyaluronic acid: dermatan sulphate weight ratio comprised between 1:0.06 and 1:0.80, preferably between 1:0.10 and 1:0.50. The especially preferred hyaluronic acid:dermatan sulphate weight ratio is 1:0.25.

In another also preferred embodiment of the composition for use, the composition further comprises collagen hydrolysate. The hyaluronic acid, dermatan sulphate and collagen hydrolysate are preferably present at a hyaluronic acid:dermatan sulphate:collagen hydrolysate weight ratio comprised between 1:0.06:0.03 and 1:0.80:0.40, preferably between 1:0.10:0.05 and 1:0.50:0.20. The especially preferred hyaluronic acid:dermatan sulphate:collagen hydrolysate weight ratio is 1:0.20:0.10. Another especially preferred weight ratio is 1:0.25:0.10.

The composition for use is preferably adapted for oral or parenteral administration. Oral administration is most preferred.

Another aspect of the invention is a non-therapeutic use of a composition comprising hyaluronic acid and dermatan sulphate as a food, functional food or food supplement for reducing overweight, reducing food intake, inducing the feeling of satiety, reducing appetite, reducing body weight, preventing weight gain, reducing body fat or reducing fat formation in a mammal. Preferably, the mammal has overweight. The use is preferably for reducing food intake. Another aspect of the invention is the use of a previously defined composition as a food, functional food or food supplement for reducing visceral and/or subcutaneous fat accumulation.

In another also preferred embodiment, the mammal is a human.

Another aspect of the invention is another non-therapeutic use of a composition comprising hyaluronic acid and dermatan sulphate as a slimming product. Said composition is preferably in the form of a food, a functional food or a food supplement. The food can be a dietetic food.

In another preferred embodiment of said non-therapeutic uses, hyaluronic acid and dermatan sulphate are present at a hyaluronic acid: dermatan sulphate weight ratio comprised between 1:0.06 and 1:0.80, preferably between 1:0.10 and 1:0.50. The especially preferred hyaluronic acid:dermatan sulphate weight ratio is 1:0.25.

In another also preferred embodiment of said non-therapeutic uses, the composition further comprises collagen hydrolysate. The hyaluronic acid, dermatan sulphate and collagen hydrolysate are preferably present at a hyaluronic acid:dermatan sulphate:collagen hydrolysate weight ratio comprised between 1:0.06:0.03 and 1:0.80:0.40, preferably between 1:0.10:0.05 and 1:0.50:0.20. The especially preferred hyaluronic acid:dermatan sulphate:collagen hydrolysate weight ratio is 1:0.20:0.10. Another especially preferred weight ratio is 1:0.25:0.10.

In the present invention, the term "comprises" must be interpreted such that it also includes the case of "only consists of" and "essentially consists of".

In the present invention, when referring to hyaluronic acid and dermatan sulphate, any salt thereof, for example sodium, potassium or calcium salt, is included.

The hyaluronic acid and dermatan sulphate of the compositions of the present invention are preferably in the form of a sodium salt.

In the present invention, the mean molecular weights of hyaluronic acid, dermatan sulphate and chondroitin sulphate have been determined by gel permeation chromatography (GPC).

In the present invention, the abbreviation HA means Hyaluronic Acid, the abbreviation DS means Dermatan Sulphate, the abbreviation CH means Collagen Hydrolysate and the abbreviation CS means Chondroitin Sulphate.

In the present invention, the following terms have the indicated meaning:
"Functional food" refers to a food that, besides its basic nutritional role from the material and energy viewpoint, is able to provide a health benefit because it contains one or more biologically active components or a combination of biologically active components (for example, a composition of the invention). The functional food is part of an individual's diet.

"Food supplement" refers to a diet supplement containing one or more nutrients or other biologically active components (for example, a composition of the invention) in concentrated form with a nutritional or physiological effect that is beneficial for health. It is administered in the form of tablets, capsules or in any other dose form.

"Medical food" refers to a food administered to a patient under the instructions and supervision of a doctor. Said food is specific for the nutritional requirements of a patient having a certain disease, discomfort or disorder. It is used in the United States and is usually presented in the form of a food, although it can also be found in a dose form. The equivalent to a "medical food" in Europe is a "dietetic food for special medical uses", which is a food intended for a special diet, that has been specially prepared or formulated to completely or partially satisfy the dietary needs of patients whose capability of ingesting, digesting, absorbing, metabolizing or excreting normal foods or certain nutrients of said foods or metabolites is limited or deficient, or is altered, or of patients who require other clinically determined nutrients, whose dietary treatment is not possible by only modifying the normal diet with other foods intended for a special diet, or by means of both. It may or may not be in dose form.

"Dietetic food" refers to a food that is indicated for a specific nutritional purpose due to its composition.

"Metabolic syndrome" refers to the co-occurrence of overweight or obesity and several additional cardiovascular risk factors, such as insulin resistance and hyperinsulinemia, glucose intolerance, dyslipidemia, and hypertension.

"Inducing the feeling of satiety" refers to inducing that the individual feels satisfied and not hungry, hunger being understood as the need for an immediate intake of food.

"Maintaining normal blood cholesterol levels" refers to maintaining total circulating cholesterol levels below 200 mg/dL, maintaining circulating LDL cholesterol levels below 100 mg/dL and maintaining circulating HDL cholesterol levels above 40 mg/dL.

"LDL cholesterol" refers to the cholesterol bound to the low-density lipoproteins, also known as "bad cholesterol".

"HDL cholesterol" refers to the cholesterol bound to high-density lipoproteins, also known as "good cholesterol".

"Hypercholesterolemia" refers to when the blood total cholesterol and/or LDL cholesterol levels are high (blood total cholesterol levels above 200 mg/dL; blood LDL cholesterol levels above 100 mg/dL).

"Maintaining normal blood triglyceride levels" refers to maintaining triglyceride levels below 150 mg/dL.

"Hyperleptinemia" refers to when blood or synovial fluid leptin levels are high.

"Parenteral route" refers to when medicaments or nutrients are introduced in the body without being absorbed by the gastrointestinal tract. The most frequently used parenteral routes are the intravenous, subcutaneous and intramuscular routes.

"Anti-cellulite product" refers to a product for reducing subcutaneous fat that is accumulated in the form of nodules in certain areas of the body.

The hyaluronic acid and dermatan sulphate of the compositions of the present invention can be obtained by methods of extraction from tissues of birds or of mammals or by biotechnology.

The mean molecular weights of hyaluronic acid and of dermatan sulphate of the compositions of the present invention can vary depending on the production method. Preferably, the mean molecular weight of hyaluronic acid is comprised between 300,000 and 2,000,000 daltons, more preferably between 800,000 and 1,000,000 daltons, and the mean molecular weight of dermatan sulphate is comprised between 10,000 and 50,000 daltons.

The hyaluronic acid of the compositions of the present invention can be obtained by means of extraction from tissues of birds or of mammals, for example from vitreous humour, the skin of a mammal, the umbilical cord or crests of birds, or by the fermentation of microorganisms (for example *Streptococcus*), following methods described in the literature (D.A. Swann, Biochim. Biophys. Acta 156, 17-30 (1968); patent US 4,780,414).

The hyaluronic acid of the compositions of the present invention is preferably a commercial product available at www.bioiberica.com. It is obtained from cockscombs which are digested with a proteolytic enzyme once they are ground up. The enzyme is subsequently deactivated by means of heating; it is filtered, the dermatan sulphate is removed and the hyaluronic acid, which is made anhydrous, is precipitated, dried and ground up. Said hyaluronic acid in the form of a sodium salt has a minimum purity of 90%, determined by the glucuronic acid content, and a mean molecular weight comprised between 800,000 and 1,000,000 daltons.

The dermatan sulphate of the compositions of the present invention can be obtained from tissues of birds or of mammals, for example from porcine or bovine mucosa, the crests of birds or the skin of a mammal, following methods described in the literature (N. Volpi, Anal. Biochem. 218, 382-391 (1994); patent EP 238994).

The dermatan sulphate of the compositions of the present invention is preferably obtained from cockscombs in the method for obtaining hyaluronic acid. Once the dermatan sulphate in the form of a complex is separated, the complex is broken by means of ionic strength, precipitated, made anhydrous, dried and ground up. The dermatan sulphate of porcine mucosa marketed by the company Sigma-Aldrich Química (reference: C3788) can also be used. The dermatan sulphate in the form of a sodium salt that comes from cockscombs has a minimum purity of 90%, determined by photometric titration, and a mean molecular weight comprised between 10,000 and 50,000 daltons.

The collagen hydrolysate of the compositions of the present invention can be obtained from the skin of a mammal or from cockscombs following methods described in the literature ("Final Report on the Safety Assessment of Hydrolyzed Collagen", Journal of the American College of Toxicology 4, no. 5, 199-221, Mary Ann Liebert, Inc., Publishers, (1985)).

The compositions of the present invention can be prepared by mixing their components at the desired proportions. Compositions containing hyaluronic acid and dermatan sulphate, or hyaluronic acid, dermatan sulphate and collagen hydrolysate (low molecular weight peptides and amino acids) can therefore be prepared.

The compositions of the present invention can also be obtained directly from the tissues of birds or of mammals by means of an extraction method. Therefore, for example, the method can start from frozen cockscombs which are digested with a proteolytic enzyme once they are ground up. The enzyme is subsequently deactivated by means of heating, it is filtered and precipitated with solvents. It is then filtered, washed and dried. Grinding can finally be performed. The obtained product comprises hyaluronic acid, dermatan sulphate and collagen hydrolysate (in the form of low molecular weight peptides and amino acids). By slightly modifying this method compositions comprising hyaluronic acid and dermatan sulphate can be obtained, but not collagen hydrolysate. The presence of the three components (HA, DS and CH) or of only two (HA and DS) and their proportion will depend on the production method (type of enzyme, temperature, reaction time and purification process).

The most preferred composition of the invention comprising hyaluronic acid, dermatan sulphate and collagen hydrolysate is the composition comprising 65% hyaluronic acid (HA), 13.40% dermatan sulphate (DS) and 6.56% collagen hydrolysate (CH) (HA:DS:CH weight ratio of 1:0.20:0.10).

In the present invention, the glycosaminoglycan chondroitin sulphate has been used only for comparative purposes. Chondroitin sulphate has a polymer structure characterised by a repeating disaccharide, made up of *N*-acetyl-D-galactosamine and D-glucuronic acid. Most *N*-acetyl-D-galactosamine residues are sulphated. It can be obtained by means of proteolytic enzyme digestion of cartilage tissues of animals, for example bovine or porcine livestock tracheas or cartilaginous fish skeletons. The chondroitin sulphate that comes from cartilage tissue is primarily found in two isomeric forms, 4-chondroitin sulphate and 6-chondroitin sulphate. The mean molecular weight of the chondroitin sulphate can vary depending on its origin and/or on the production method used. It generally has a mean molecular weight comprised between 10,000 and 70,000 daltons.

The chondroitin sulphate in the form of a sodium salt used for comparative purposes in Example 1 of the present invention is a commercial product available at www.bioiberica.com with a minimum purity of 98%, determined by photometric titration, and a mean molecular weight comprised between 14,000 and 18,000 daltons. It is obtained from bovine cartilage.

To use the compositions of the present invention in the treatment or prevention of overweight, obesity, insulin resistance, type 2 diabetes, fatty liver or dyslipidemia in a mammal, said compositions are formulated in suitable pharmaceutical compositions using conventional techniques and excipients or carriers, such as those described in Remington: The Science and Practice of Pharmacy 2000, edited by Lippincott Williams and Wilkins, 20th editi*on, Philadelphia.* The pharmaceutical compositions comprise a therapeutically effective amount of a composition of the present invention and at least one excipient that is pharmaceutically acceptable for administering to the patient. Said pharmaceutical compositions can be administered to the patient at required doses. The pharmaceutical compositions can be administered through different routes, for example, oral, intravenous, subcutaneous, intramuscular, sublingual, intradermal, nasal or topical route. The pharmaceutical compositions of the invention include a therapeutically effective amount of a composition of the present invention, said amount depending on many factors, such as for example, the patient's physical condition, age, sex, administration route, administration frequency or seriousness of the disease. Furthermore, it will be understood that said dosage of the composition of the invention can be administered in single- or multiple-dose units to provide the desired therapeutic effects.

The pharmaceutical compositions of the invention will generally be in solid form, liquid form or gel form. Powders, pellets, tablets, dispersible granules, capsules, cachets, tablets, lyophilisates and suppositories are included among the pharmaceutical preparations in solid form that can be prepared according to the present invention. Solutions, suspensions, emulsions, syrups, elixirs, drinkable vials and herbal teas are included among the preparations in liquid form. Preparations of solid forms which are to be converted into preparations in liquid form immediately before use are also contemplated. Said liquid forms include solutions, suspensions and emulsions.

The compositions for use wherein the treatment or prevention of dyslipidemia results in reducing blood cholesterol, maintaining normal blood cholesterol levels, reducing blood triglycerides or maintaining normal blood triglyceride levels, as well as the compositions for use wherein the treatment or prevention of insulin resistance or type 2 diabetes results in increasing insulin sensitivity, are prepared in dose forms containing a composition of the invention; dietetic foods for those special medical uses are prepared in dose forms containing a composition of the invention and additives used in nutrition; and medical foods or dietetic foods for special medical uses are prepared in non-dose forms containing a composition of the invention and nutrients or foods. The dietetic food for special medical uses can be in the form of tablets, capsules, solutions, suspensions or sachets or also as a food for the special diet of patients. Medical food is usually presented in the form of a food for feeding patients, although it also can be found in dose form.

To use the compositions of the present invention in reducing food intake, inducing the feeling of satiety, reducing appetite, reducing body weight, preventing weight gain, reducing body fat, reducing fat formation, in a mammal, or as a slimming product or food supplements are prepared in dose forms containing a composition of the invention and additives used in nutrition, or foods or functional foods are prepared, adding the compositions of the invention to the foods that are part of the diet. The food supplement can be in the form of tablets, capsules, solutions, suspensions or sachets. The functional food can be in the form of yogurts, milk, fermented milk, fruit juices, vegetable juices, soups, dehydrated foods, biscuits or baby foods.

### Brief Description of the Drawings

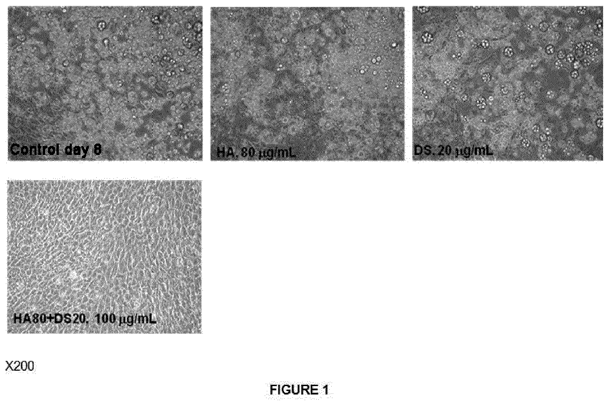

Figure 1 shows images taken by phase contrast microscopy of mouse embryonic fibroblasts (MEFs) cultured for 8 days in growth medium containing: carrier (water, Control); hyaluronic acid (HA) at a final concentration (f.c.) of 80 µg/mL; dermatan sulphate (DS) at an f.c. of 20 µg/mL; or composition of the invention HA80+DS20 at an f.c. of 100 µg/mL. Droplets of intracellular fat refringent under phase contrast microscopy are seen in all the cultures except the one treated with HA80+DS20 (x200 magnification).

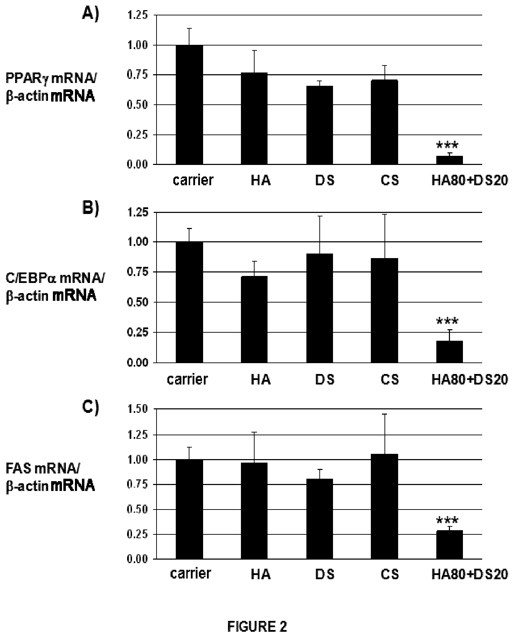

Figure 2 shows the expression levels of messenger RNAs (mRNAs) for the adipogenic transcription factors PPARgamma (A) and C/EBPalpha (B) and for the lipogenic enzyme fatty acid synthase (FAS) (C) in MEFs cultured for 8 days in growth medium supplemented with: carrier (water); HA at an f.c. of 80 µg/mL; DS at an f.c. of 20 µg/mL; CS at an f.c. of 20 µg/mL or composition of the invention HA80+DS20 at an f.c. of 100 µg/mL. The mRNA levels for beta-actin were used as an internal control. The results are expressed on a per unit basis of the expression level in the control cells treated with carrier, and they are the mean ± SEM of at least six samples for each tested condition, distributed in two independent experiments. ***; P= 0.001 vs. carrier, t-test.

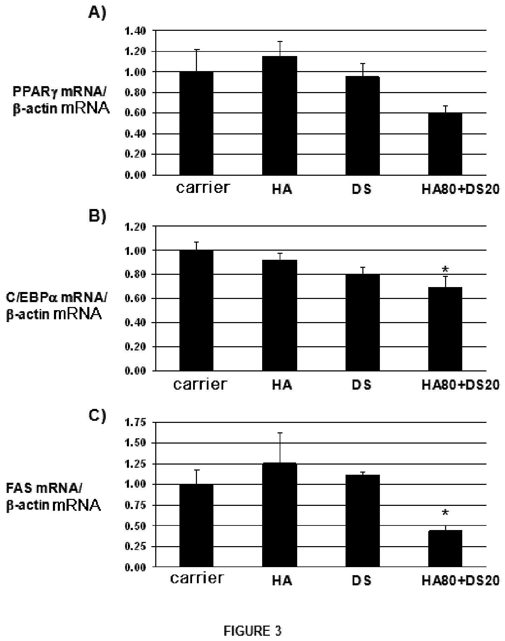

Figure 3 shows the expression levels of messenger RNAs (mRNAs) for the adipogenic transcription factors PPARgamma (A) and C/EBPalpha (B) and for the lipogenic protein FAS (C) in MEFs after 8 days of differentiation in medium containing an adipogenic hormone cocktail supplemented with: carrier (water); HA at an f.c. of 80 µg/mL; DS at an f.c. of 20 µg/mL; or composition of the invention HA80+DS20 at an f.c. of 100 µg/mL. The mRNA levels for beta-actin were used as an internal control. The results are expressed on a per unit basis of the expression level in the control cells treated with carrier, and they are the mean ± SEM of triplicates for each treatment. *; P<0.05 vs. carrier, t-test.

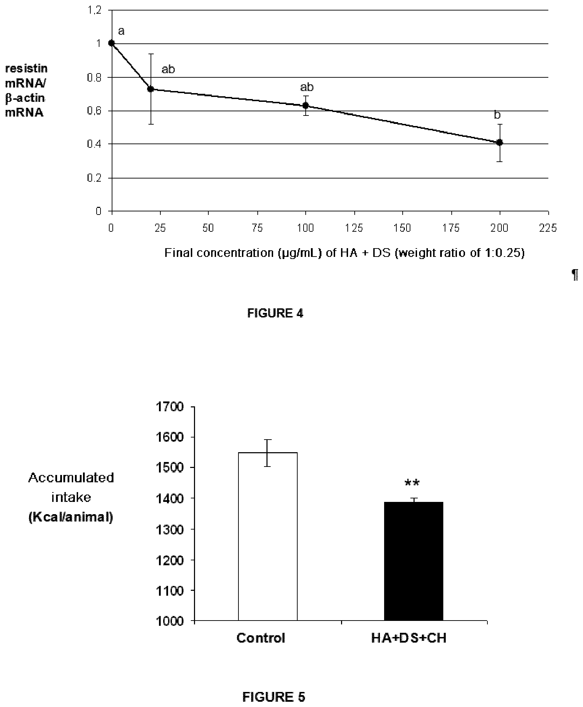

Figure 4 shows the expression levels of resistin in MEFs after 8 days of differentiation in adipogenic medium in the presence of different final concentrations of HA+DS at a HA:DS weight ratio of 1:0.25. The mRNA levels for beta-actin were used as an internal control. The results are expressed on a per unit basis of the expression level in the control cells treated with carrier, and they are the mean ± SEM of four experiments performed in triplicate. The different letters (a, b) imply significant changes according to a single-factor ANOVA (P<0.05).
Figure 5 shows the accumulated energy intake of mice after 143 days of treatment, by oral route, with the carrier (water, Control group), or with a composition of the invention, at a dose of 0.45 mg/mouse/day (HA+DS+CH group). The depicted data corresponds to the mean ± SEM of 8 animals per group, distributed in 2 cages with 4 animals each. **; P<0.01 HA+DS+CH vs. Control, t-test.

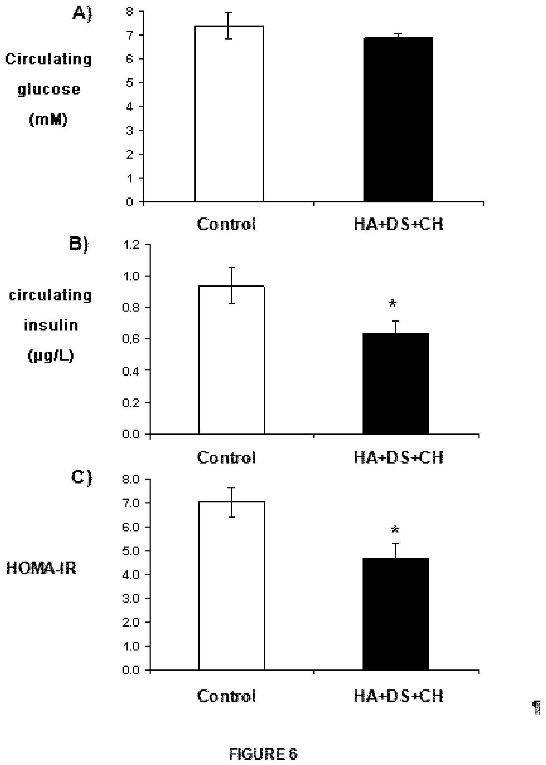

Figure 6 shows the circulating glucose levels (A), circulating insulin levels (B) and HOMA-IR index (C) in mice treated with the carrier (water, Control group) or with a composition of the invention, at a dose of 0.45 mg/mouse/day (HA+DS+CH group), by oral route. The analyses were conducted after 88 days of treatment, after subjecting the animals to a 6-hour fast. The data corresponds to the mean ± SEM of 7-8 animals per group. *; P<0.05 HA+DS+CH vs. Control, t-test.

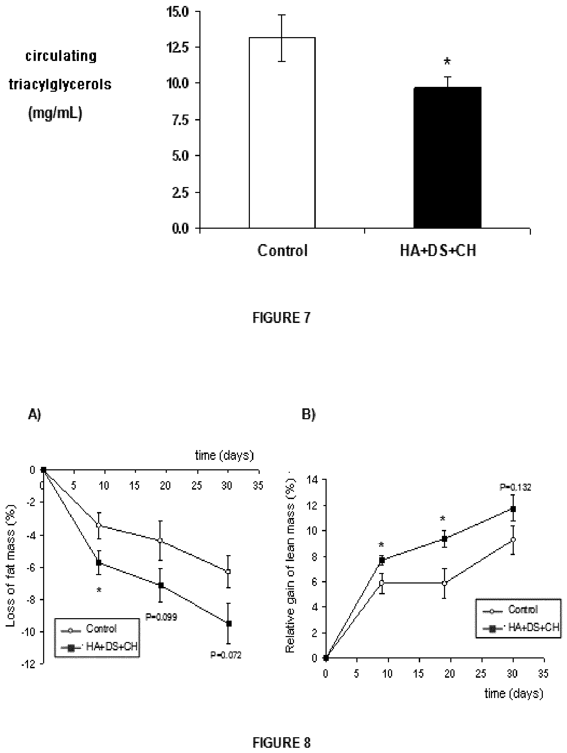

Figure 7 shows the circulating triacylglycerol levels in the fed state in mice after 143 days of treatment with the carrier (Control group) or with a composition of the invention, at a dose of 0.45 mg/mouse/day (HA+DS+CH group). The data corresponds to the mean ± SEM of 7-8 animals per group. *; P<0.05 HA+DS+CH vs. Control, t-test.
Figure 8 shows the evolution of the loss of body fat mass (A) and the relative gain of lean body mass (B) in mice with dietary obesity in the slimming phase, treated with the carrier (Control group) or with a composition of the invention, at a dose of 3 mg/mouse/day (HA+DS+CH group), daily by oral route. The results are the mean ± SEM of 7-8 animals per group. *; P<0.05 HA+DS+CH vs. Control at respective time, t-test.

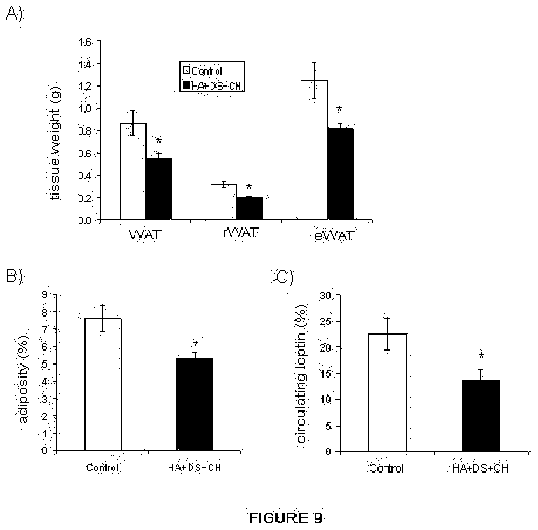

Figure 9 shows the weight of inguinal white adipose tissue (iWAT) depot, retroperitoneal white adipose tissue (rWAT) depot and epididymal white adipose tissue (eWAT) depot (A), the body adiposity index (B) and the circulating leptin concentration (C) in obese mice exposed for 32 days to slimming conditions and treatment with the carrier (Control group) or with a composition of the invention, at a dose of 3 mg/mouse/day (HA+DS+CH group). The adiposity index for each animal was defined as the sum of the weight of its WAT depots expressed as a percentage of body weight. The results are the mean ± SEM of 7 animals per group. *; P<0.05 HA+DS+CH vs. Control, t-test.

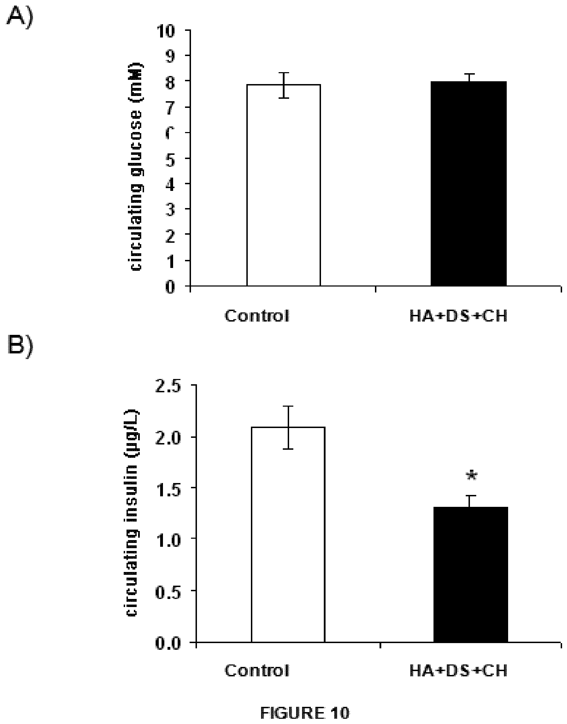

Figure 10 shows the circulating glucose levels (A) and circulating insulin levels (B) in the fed state in obese mice exposed for 32 days to slimming conditions and treatment with the carrier (Control group) or with a composition of the invention, at a dose of 3 mg/mouse/day (HA+DS+CH group). The results are the mean ± SEM of 6-7 animals per group. *; P<0.05 HA+DS+CH vs. Control, t-test.

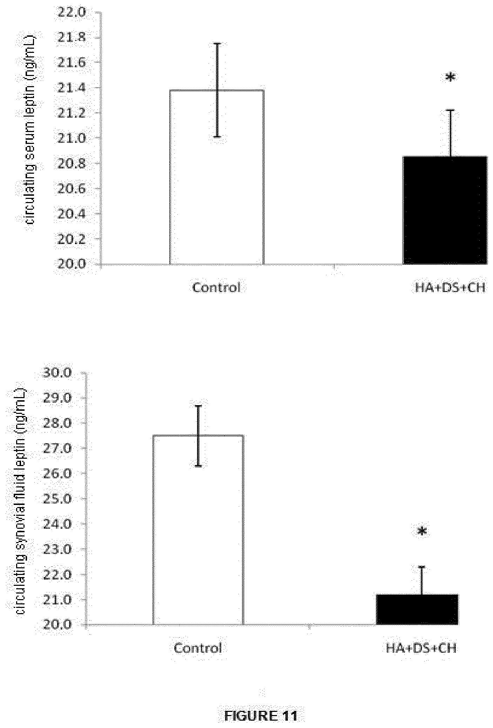

Figure 11 shows the circulating serum and synovial fluid leptin concentration in obese people treated with excipients (Control) or with a composition of the invention (HA+DS+CH), at a dose of 80 mg/person/day.

### Detailed Description of the Preferred Embodiments

The following examples are provided for illustrative purposes and do not represent a limitation to the scope of the present invention.

### Example 1: Study of the efficacy and synergy of a composition of the present invention in inhibiting spontaneous adipogenesis of mouse embryonic fibroblasts. Comparison with hyaluronic acid, dermatan sulphate and chondroitin sulphate

Adipogenesis is the process of differentiation of fat storage cells (adipocytes) from precursor cells. Obesity is often correlated with the presence of an excessive number of adipocytes in fat depots. The objective of the study was to determine if there was a synergistic effect between the components of a composition of the present invention to suppress adipogenesis. To that end, the potential of a combination of hyaluronic acid and dermatan sulphate at a HA:DS weight ratio of 1:0.25 (HA80+DS20), and that of the individual components thereof in inhibiting intracellular lipid accumulation and the expression of adipocyte marker genes in primary mouse embryonic fibroblast (MEFs) cultures kept in growth medium were determined. A comparison with glycosaminoglycan chondroitin sulphate was also carried out. MEFs are culturable, multipotent cells that are able to differentiate into different mesenchymal cell types (adipocytes, chondrocytes, osteoblasts) depending on the hormonal stimulation they receive. Some studies have reported that the serum of the culture medium can induce spontaneous adipogenesis in multipotent mesenchymal cells such as MEFs (L. Wu et al., J. Cell Mol. Med. 14, 922-932 (2010)).

### Materials and methods

MEFs obtained from embryos of 13 days gestational age were cultured in monolayer in growth medium consisting of AmnioMAX™-C100 (Invitrogen, Carlsbad, CA, USA) basal medium supplemented with AmnioMAX™-C100 supplement (7.5%), foetal bovine serum (7.5%) and antibiotics, in 12-well culture plates of 1 mL capacity/well, following a previously described protocol (J.B. Hansen et al., Proc. Natl. Acad. Sci. USA 101, 4112-4117 (2004)). Two days after reaching confluence, which is considered day 0 of culture, the cells were exposed to: carrier (water); hyaluronic acid (HA) at a final concentration (f.c.) of 80 µg/mL; dermatan sulphate (DS) at an f.c. of 20 µg/mL; chondroitin sulphate (CS) at an f.c. of 20 µg/mL; or composition of the present invention HA80+DS20 at an f.c. of 100 µg/mL, containing 80 µg of HA and 20 µg of DS per mL. The culture medium in contact with the cells was changed every two days (day 2, 4 and 6 of culture) with fresh medium, always with the aforementioned supplements. The cells were collected on day 8 of culture, after being examined and photographed under phase contrast microscopy to view their shape and degree of lipid accumulation. The mRNA levels of the genes related to adipocyte differentiation PPARgamma, C/EBPalpha and fatty acid synthase (FAS) were determined by real-time PCR from the total RNA extracted from the cells with Trizol reagent and reverse-transcribed with reverse transcriptase, and using the mRNA of beta-actin as an internal control. Specific primer pairs for each gene were designed using the Primer 3 software (Whitehead Institute for Biomedical Research, Cambridge, MA, USA), and their specificity was checked by means of the ENTREZ and BLAST databases (National Center for Biotechnology Information, Bethesda, MD, USA). The primers were produced by Sigma (Madrid, Spain). The statistical significance of differences with respect to treatment with the carrier was analyzed by means of Student's t-test. The significance threshold was established at P<0.05.

### Results

The observation of the cells at day 8 of culture showed the presence of a number of clusters of cells spontaneously differentiated into adipocytes filled with fat droplets, refringent under phase contrast microscopy, in the cultures treated with carrier, HA80, and DS20, but not in the cultures treated with the composition of the invention HA80+DS20 (Figure 1).

According to the foregoing, at the molecular level, the expression of the PPARgamma, C/EBPalpha and FAS adipocyte marker genes was drastic and significantly decreased in cells treated with the composition of the invention HA80+DS20, but not in cells treated with the components of the composition separately or with CS (Figure 2).

These results indicate that the combination of hyaluronic acid and dermatan sulphate inhibits spontaneous adipogenesis in MEFs in culture with a synergistic effect, which exceeds the sum of the effects of the individual components of the combination separately. Therefore, the composition of the invention can be of great use in reducing fat formation and in the treatment or prevention of overweight, obesity and associated metabolic complications.

### Example 2: Study of the efficacy and synergy of a composition of the present invention in inhibiting the expression of adipocyte markers during hormone-induced adipogenesis of mouse embryonic fibroblasts. Comparison with hyaluronic acid and dermatan sulphate

The objective of the study was to compare the potential of a combination of hyaluronic acid and dermatan sulphate at a HA:DS weight ratio of 1:0.25 (HA80+DS20), and of the individual components thereof in affecting adipogenesis in mouse embryonic fibroblast (MEF) cultures hormonally stimulated to differentiate into adipocytes, and to determine if there was a synergistic effect between hyaluronic acid and dermatan sulphate to this respect.

### Materials and methods

MEFs obtained from embryos of 13 days gestational age were cultured in monolayer in 12-well culture plates having a 1 mL capacity/well, as described in Example 1. Two days after reaching confluence (day 0), the cells were stimulated by exposing them for 48 hours to a standard adipogenic hormone cocktail containing dexamethasone, methylisobutylxanthine, insulin and rosiglitazone. The cells subsequently received fresh growth medium supplemented with insulin and rosiglitazone every two days (day 2, 4 and 6). The differentiation process was carried out in cells exposed from day 0 to: carrier (water); HA at a final concentration (f.c.) of 80 µg/mL; DS at an f.c. of 20 µg/mL; or composition of the present invention HA80+DS20 at an f.c. of 100 µg/mL, containing 80 µg of HA and 20 µg of DS per mL. The cells were collected on day 8 of culture. The mRNA levels of selected genes were determined by real-time PCR, as described in Example 1.

### Results

Expression of the PPARgamma, C/EBPalpha and FAS adipocyte marker genes decreased in cells exposed to the composition of the invention HA80+DS20 during the adipogenic process, but not in those exposed to the individual components of the composition, HA or DS, separately (Figure 3).

These results indicate that the combination of HA and DS exerts a synergistic anti-adipogenic effect even under environmental conditions (presence of a pro-adipogenic hormone cocktail) that enhance this process. As a result, the composition of the invention can be used in the treatment or prevention of overweight, of obesity and of the metabolic complications associated therewith, as well as in reducing fat formation.

### Example 3: Study of the efficacy of a composition of the present invention in inhibiting the expression of resistin, an adipokine related to insulin resistance, in MEF-derived adipocytes

One of the most common metabolic complications in obesity is insulin resistance, which is closely associated with type 2 diabetes. The objective was to study the effect of different concentrations of a combination of hyaluronic acid and dermatan sulphate at, at a HA:DS weight ratio of 1:0.25 (HA+DS) on the expression of secretion proteins of adipocytes known to be implicated as factors that enhance insulin resistance, particularly resistin, in adipose cells in culture. The effect of hyaluronic acid and of dermatan sulphate separately was also determined in the same study.

### Materials and methods

MEFs obtained from embryos of 13 days gestational age were cultured in monolayer until reaching confluence and exposed to a standard adipogenic hormone cocktail as described in Example 2, in the presence of: carrier (water); HA at an f.c. of 80 µg/mL; DS at an f.c. of 20 µg/mL; composition of the invention HA16+DS4 at an f.c. of 20 µg/mL, containing 16 µg of HA and 4 µg of DS per mL; composition of the invention HA80+DS20 at an f.c. of 100 µg/mL, containing 80 µg of HA and 20 µg of DS per mL; or composition of the invention HA160+DS40 at an f.c. of 200 µg/mL, containing 160 µg of HA and 40 µg of DS per mL. The cells were collected on day 8 of culture. Expression levels of the resistin gene were determined by real-time PCR, as described in Example 1.

### Results

Exposure to the compositions of the invention HA16+DS4 at a final concentration of 20 µg/mL, HA80+DS20 at a final concentration of 100 µg/mL or HA160+DS40 at a final concentration of 200 µg/mL reduced the expression of resistin after 8 days of MEF adipocyte differentiation in a dose-dependent manner (Figure 4). The effect of the compositions of the invention on the expression of the resistin gene was not reproduced by the individual components HA80 and DS20 when they were tested separately. The reduction of the expression of resistin is correlated with lower insulin resistance, and therefore with greater insulin sensitivity.

### Example 4: Effect of supplementation with a composition of the present invention on the reduction of weight gain and of energy intake in mice

The objective was to study the effect of supplementation by oral route with a composition of the present invention (HA+DS+CH) on the evolution of biometric parameters *in vivo* in mice.

A composition containing 65% hyaluronic acid (HA), 13.40% dermatan sulphate (DS) and 6.56% collagen hydrolysate (CH) (HA:DS:CH weight ratio of 1:0.20:0.10) was used.

### Materials and methods

C57BL6/J male mice (Charles River Laboratories, Barcelona, Spain) 6 weeks of age at the start of the experiment were housed at 22°C with a 12-hour light/dark period, under *ad libitum* feeding conditions with a standard defined diet (manufactured by Research Diets, Inc, New Brunswick, NJ, USA) containing 3.85 Kcal/g of diet. At the start of the experiment, two experimental groups were established (n=8 animals per group), that received the carrier (water, Control group) or a composition of the present invention (HA+DS+CH), at a dose of 0.45 mg/mouse/day (HA+DS+CH group; HA:DS:CH weight ratio of 1:0.20:0.10), daily by oral route for 143 days. The body weight and the energy intake of the animals were tracked for the 143 days of treatment. The statistical significance of the observed effects was analyzed by means of Student's t-test. The significance threshold was established at P<0.05.

### Results

The animals treated with the composition of the invention HA+DS+CH tended to gain less weight and, at the end of the experiment, their body weight was 5% lower than that of the controls (mean±SEM: initial weights: Control group, 22.5±0.8 g; HA+DS+CH group, 22.5±0.7 g; final weights: Control group, 29.0±1.0 g; HA+DS+CH group, 27.5±1.0 g).

The accumulated energy intake during the 143 days of the experiment was 11% less in animals treated with HA+DS+CH than in animals of the Control group to which the carrier was administered (P<0.05) (Figure 5).

The *in vivo* results obtained allow asserting that the compositions of the present invention show efficacy in the treatment or prevention of overweight and obesity, in reducing body weight, in reducing weight gain, in reducing food intake and appetite, in preventing weight gain and in inducing the feeling of satiety.

### Example 5: Effect of supplementation by oral route with a composition of the present invention on parameters related to insulin sensitivity in mice

There is a connection between obesity and loss of insulin sensitivity.

The objective was to study the effect of supplementation by oral route with a composition of the present invention (HA+DS+CH) on *in vivo* insulin sensitivity in mice.

A composition containing 65% hyaluronic acid (HA), 13.40% dermatan sulphate (DS) and 6.56% collagen hydrolysate (CH) (HA:DS:CH weight ratio of 1:0.20:0.10) was used.

### Materials and methods

The same experimental groups as in Example 4 were used. After 88 days of treatment, the animals from both the Control and HA+DS+CH groups were subjected to a 6-hour fast after which blood was taken for determining the circulating glucose level (by Roche's Accu-Check system) and circulating insulin level (by means of an ELISA kit distributed by DGR Instruments GnbH). The HOMA-IR (homeostatic model assessment for insulin resistance) index, which is a well-accepted index indicative of insulin sensitivity, was calculated for each animal using the formula of Matthews *et al.* (D.R. Matthews et al., Diabetologia 28, 412-419 (1985)): HOMA-IR= fasting glucose (mM) x fasting insulin (mU/L)/22.5. The lower the HOMA-IR index, the lower the insulin resistance and therefore the higher the sensitivity to this hormone. This index has been validated in humans and mice (S. Lee et al. Am. J. Physiol. Endocrinol. Metab. 294, E261-70 (2008)).

### Results

Animals supplemented with HA+DS+CH at the same blood glucose level showed lower insulinemia, and therefore a lower HOMA-IR index as well, with respect to the control animals (Figure 6). These results indicate that insulin sensitivity is greater in the group supplemented with the composition of the invention comprising hyaluronic acid, dermatan sulphate and collagen hydrolysate (HA+DS+CH) than in the Control group. Insulin sensitivity impairment is considered to be one of the main factors that triggers the development of type 2 diabetes, together with pancreatic beta cell dysfunction (D. LeRoith, Am. J. Med. 113 (Suppl 6A), 3S-11S (2002)).

### Example 6: Effect of supplementation by oral route with a composition of the present invention on triglyceride levels in mice

Hypertriglyceridemia is a dyslipidemia that is present in many people with overweight or obesity.

The objective of the study was to determine the effect of supplementation with a composition of the present invention (HA+DS+CH) by oral route on circulating triglyceride (triacylglycerol) levels *in vivo* in mice fed with an obesity-inducing high-fat diet.

A composition containing 65% hyaluronic acid (HA), 13.40% dermatan sulphate (DS) and 6.56% collagen hydrolysate (CH) (HA:DS:CH weight ratio of 1:0.20:0.10) was used.

### Materials and methods

The same experimental groups as in Example 4 were used. After 143 days of treatment, the animals of both the Control and HA+DS+CH groups were sacrificed by decapitation. Serum was prepared from blood of the neck and circulating serum triglyceride levels were determined by means of a commercial colorimetric enzymatic assay (Sigma).

### Results

After 143 days of supplementation, the animals supplemented with the composition of the invention comprising hyaluronic acid, dermatan sulphate and collagen hydrolysate (HA+DS+CH) showed lower triglyceridemia than the controls to which the carrier had been administered (Figure 7). As a result, the compositions of the present invention can be of great use in the treatment or prevention of dyslipidemia and in reducing blood triglycerides.

### Example 7: Effect of supplementation by oral route with a composition of the present invention on reversing obesity, leptin concentration and insulin concentration in obese mice

Both leptin and insulin resistance are strongly related to obesity. The objective was to study the loss of weight and fat and the reduction of circulating leptin and insulin in obese mice when they were treated with a composition of the present invention (HA+DS+CH) by oral route.

A composition containing 65% hyaluronic acid (HA), 13.40% dermatan sulphate (DS) and 6.56% collagen hydrolysate (CH) (HA:DS:CH weight ratio of 1:0.20:0.10) was used.

### Materials and methods

C57BL6/J male mice 25 weeks of age in which dietary obesity had previously been induced by feeding them with a high-fat diet starting from 6 weeks of life were used. At the start of the experiment, the mice showed 22% excess weight with respect to animals of the same age kept under a standard diet. At the start of the experiment, the obese mice were switched to a normal-fat diet and distributed into two groups that received the carrier (water, 60 µl, Control group) or a composition of the present invention (HA+DS+CH), at a dose of 3 mg/mouse/day (n=7-8 animals/group) (HA+DS+CH group; HA:DS:CH weight ratio of 1:0.20:0.10) daily by oral route. Body weight and body composition were regularly monitored in both the Control and HA+DS+CH groups by means of a non-invasive magnetic resonance system (ECHO-MRI). The animals were sacrificed by decapitation 32 days after the start of reversing to a standard diet, blood and tissues were collected, and the weight of the adipose tissues were recorded. The statistical significance of the observed effects was analyzed by means of Student's t-test. The significance threshold was established at P<0.05.

### Results

The animals treated with HA+DS+CH tended to lose a higher percentage of weight and to lose it faster than those of the Control group; this trend reached statistical significance after 11 days of reversing to the normal-fat diet, at which point the animals of the Control group had lost 9.5% of their initial body weight, and the animals treated with HA+DS+CH had lost 12.8%. Furthermore, the animals treated with HA+DS+CH showed a faster and higher percentage-based loss of fat mass than the animals of the Control group (Figure 8A), which was correlated with a greater relative increase of their lean mass (Figure 8B).

At the end point of the experiment, the adiposity index (sum of the weights of the adipose tissue depots as percentage of body weight) of the animals treated with HA+DS+CH was 30% less than that of the animals of the Control group (Figure 9B). All the dissected fat depots (inguinal (iWAT), retroperitoneal (rWAT) and epididymal (eWAT)) were significantly smaller in the animals treated with HA+DS+CH (Figure 9A). In good correspondence with this lower adiposity, at the end point of the experiment the circulating leptin concentration was 40% less in the animals treated with HA+DS+CH (Figure 9C).

At the end point of the experiment, despite not showing blood glucose variations, the animals treated with HA+DS+CH showed lower insulinemia than the Control animals, which is indicative of greater insulin sensitivity (Figure 10).

On one hand, the present study confirms that leptin and insulin levels are high in obese/overweighed animals (see controls in Figure 9C and Figure 10B), and on the other hand, it clearly shows that the compositions of the invention aid in reversing obesity and to act on leptinemia and insulinemia at the same time.

These *in vivo* results of reversing obesity and reducing leptinemia and insulinemia allow asserting that the compositions of the present invention can be of great use in the treatment or prevention of overweight, and of obesity, and in the treatment and prevention of metabolic disorders occurring with obesity and defining metabolic syndrome.

### Example 8: Effect of supplementation by oral route with a composition of the present invention on leptinemia in obese patients

Leptin, a hormone synthesized and secreted by adipose tissue (specifically by the adipocyte), controls energy balance at the level of the hypothalamus, inhibiting food intake and stimulating energy expenditure. If there is an excess food intake, there is excess energy available which accumulates in the form of fat (adipocyte hypertrophy and hyperplasia), causing obesity. Leptin levels are high in obese people, indicating the resistance of these individuals to the effects of leptin.

The objective was, on one hand, to study leptinemia in obese people treated with a composition of the present invention (HA+DS+CH) by oral route, and on the other hand, to study how said treatment would affect the cholesterol and triglyceride levels.

A composition containing 65% hyaluronic acid (HA), 13.40% dermatan sulphate (DS) and 6.56% collagen hydrolysate (CH) (HA:DS:CH weight ratio of 1:0.20:0.10) was used.

### Materials and methods

49 obese people with a mean body mass index of 34.6±0.87 kg/m² were recruited. The participants were randomly divided into 2 groups. One group received 2 capsules containing a composition of the present invention (HA+DS+CH), at a dose of 80 mg/person/day (HA+DS+CH group; HA:DS:CH weight ratio of 1:0.20:0.10) daily by oral route. The other group received a placebo treatment consisting of administrating 2 capsules containing only the excipients used in the preceding composition daily by oral route (Control group). The participants received the products under study for a period of 90 days. At the start (day 0) and end of the study (day 90), blood samples were obtained from all the participants in order to analyze the leptin concentration, the total cholesterol and triglycerides, and synovial fluid samples in order to analyze the leptin concentration. 40 of the 49 participants who started the study completed the treatment. The statistical significance of the observed effects was analyzed by means of a covariance analysis. The significance threshold was established at P<0.05.

### Results

At the start of the study, the serum and synovial fluid leptin concentration was similar in the 2 treatment groups. After administrating the experimental products for 90 days, the participants who were treated with the composition of the invention (HA+DS+CH) experienced a significant reduction (p<0.05) in the serum leptin concentration (4%) and the synovial fluid leptin concentration (19%). However, in the people treated with the placebo product, the serum and synovial fluid leptin concentration remained stable between the start and the end of the study. As a result, significant differences (p<0.05) in the serum and synovial fluid leptin concentration (Figure 11) were detected at the end of the study between patients treated with HA+DS+CH and patients treated with placebo.

In relation to the cholesterol and triglyceride concentration in plasma, no differences were detected between the 2 treatment groups and the start of the study. After administering the experimental products for 90 days, the participants who were treated with the composition of the invention (HA+DS+CH) experienced a significant reduction in the concentration in plasma of cholesterol (8.8%; p=0.0056) and of triglycerides (19.4%; p=0.0175). However, in the people treated with the placebo product no significant differences were detected in the cholesterol and triglyceride concentration in plasma from the start to the end of the study.

These results obtained *in vivo* in obese people allow asserting that the compositions of the present invention are effective for regulating hyperleptinemia associated with excess body fat, and subsequently for the treatment of obesity and of the complications associated therewith which define metabolic syndrome.

## Claims

1. A composition comprising hyaluronic acid and dermatan sulphate for use in the treatment or prevention of obesity, insulin resistance, type 2 diabetes, fatty liver or dyslipidemia in a mammal.

2. The composition for use according to claim 1, wherein insulin resistance is associated with overweight or with obesity, type 2 diabetes is associated with overweight or with obesity, fatty liver is associated with overweight or with obesity or dyslipidemia is associated with overweight or with obesity.

3. The composition for use according to claim 1 or claim 2, wherein the dyslipidemia is hypercholesterolemia and/or hypertriglyceridemia.

4. The composition for use according to claim 1 or claim 2, wherein the treatment or prevention of dyslipidemia results in reducing blood cholesterol, maintaining normal blood cholesterol levels, reducing blood triglycerides or maintaining normal blood triglyceride levels.

5. The composition for use according to claim 1 or claim 2, wherein the treatment or prevention of insulin resistance or type 2 diabetes results in increasing insulin sensitivity.

6. The composition for use according to any one of claims 1 to 5, wherein the composition is in the form of a pharmaceutical composition or a medical food.

7. The composition for use according to any one of claims 1 to 6, wherein the mammal is obese or has overweight.

8. Non-therapeutic use of a composition comprising hyaluronic acid and dermatan sulphate as a food, functional food or food supplement, for reducing overweight, reducing food intake, inducing the feeling of satiety, reducing appetite, reducing body weight, preventing weight gain, reducing body fat or reducing fat formation in a mammal.

9. The non-therapeutic use of a composition according to claim 8, wherein the mammal has overweight.

10. Non-therapeutic use of a composition comprising hyaluronic acid and dermatan sulphate as a slimming product.

11. The non-therapeutic use of a composition according to claim 10, wherein the composition is in the form of a food, a functional food or a food supplement.

12. The composition for use according to any one of claims 1 to 7, or the non-therapeutic use of a composition according to any one of claims 8 to 11, wherein the hyaluronic acid and the dermatan sulphate are present at a hyaluronic acid:dermatan sulphate weight ratio comprised between 1:0.06 and 1:0.80, preferably between 1:0.10 and 1:0.50.

13. The composition for use according to claim 12, or the non-therapeutic use of a composition according to claim 12, wherein the hyaluronic acid:dermatan sulphate weight ratio is 1:0.25.

14. The composition for use according to any one of claims 1 to 7, claim 12 or claim 13, or the non-therapeutic use of a composition according to any one of claims 8 to 13, further comprising collagen hydrolysate.

15. The composition for use or the non-therapeutic use of a composition according to claim 14, wherein the hyaluronic acid, dermatan sulphate and collagen hydrolysate are present at a hyaluronic acid:dermatan sulphate:collagen hydrolysate weight ratio comprised between 1:0.06:0.03 and 1:0.80:0.40, preferably between 1:0.10:0.05 and 1:0.50:0.20.

16. The composition for use or the non-therapeutic use of a composition according to claim 15, wherein the hyaluronic acid:dermatan sulphate:collagen hydrolysate weight ratio is 1:0.20:0.10.

## Patentansprüche

1. Zusammensetzung umfassend Hyaluronsäure und Dermatansulphat für deren Verwendung bei der Behandlung oder Vorbeugung von Fettleibigkeit, Insulinresistenz, Diabetes Typ 2, Fettleber oder Dyslipidämie in einem Säugetier.

2. Zusammensetzung für deren Verwendung nach Anspruch 1, wobei die Insulinresistenz mit Übergewicht oder mit Fettleibigkeit assoziiert ist, der Diabetes Typ 2 mit Übergewicht oder mit Fettleibigkeit assoziiert ist, die Fettleber mit Übergewicht oder mit Fettleibigkeit assoziiert ist oder die Dyslipidämie mit Übergewicht oder mit Fettleibigkeit assoziiert ist.

3. Zusammensetzung für deren Verwendung nach Anspruch 1 oder Anspruch 2, wobei die Dyslipidämie Hypercholesterinämie und/oder Hypertriglyzeridämie ist.

4. Zusammensetzung für deren Verwendung nach Anspruch 1 oder Anspruch 2, wobei die Behandlung oder Vorbeugung von Dyslipidämie die Senkung des Cholesteringehalts im Blut, die Einhaltung normaler Blutcholesterinspiegel, die Senkung des Triglyceridgehalts im Blut oder die Einhaltung normaler Bluttriglyceridspiegel zu Folge hat.

5. Zusammensetzung für deren Verwendung nach Anspruch 1 oder Anspruch 2, wobei die Behandlung oder Vorbeugung von Insulinresistenz oder Diabetes Typ 2 die Erhöhung der Insulinempfindlichkeit zu Folge hat.

6. Zusammensetzung für deren Verwendung nach einem der Ansprüche 1 bis 5, wobei die Zusammensetzung in Form einer pharmazeutischen Zusammensetzung oder eines medizinischen Nahrungsmittels ist.

7. Zusammensetzung für deren Verwendung nach einem der Ansprüche 1 bis 6, wobei das Säugetier fettleibig ist oder Übergewicht hat.

8. Nicht therapeutische Verwendung einer Zusammensetzung umfassend Hyaluronsäure und Dermatansulphat als Nahrungsmittel, funktionelles Nahrungsmittel oder Nahrungsmittelergänzung, für die Senkung des Übergewichts, die Senkung der Nahrungsaufnahme, das Vermitteln des Sättigungsgefühls, die Senkung des Appetits, die Senkung des Körpergewichts, die Vorbeugung der Gewichtszunahme, die Senkung des Körperfetts oder die Senkung der Fettbildung.

9. Nicht therapeutische Verwendung einer Zusammensetzung nach Anspruch 8, wobei das Säugetier Übergewicht hat.

10. Nicht therapeutische Verwendung einer Zusammensetzung umfassend Hyaluronsäure und Dermatansulphat als Schlankheitsmittel.

11. Nicht therapeutische Verwendung einer Zusammensetzung nach Anspruch 10, wobei die Zusammensetzung in Form eines Nahrungsmittels, eines funktionellen Nahrungsmittels oder einer Nahrungsmittelergänzung ist.

12. Zusammensetzung für deren Verwendung nach einem der Ansprüche 1 bis 7, oder nicht therapeutische Verwendung einer Zusammensetzung nach einem der Ansprüche 8 bis 11, wobei die Hyaluronsäure und das Dermatansulphat bei einem Hyaluronsäure:Dermatansulphat-Gewichtsverhältnis vorhanden sind, welches zwischen 1:0,06 und 1:0,80, vorzugsweise zwischen 1:0,10 und 1:0,50, liegt.

13. Zusammensetzung für deren Verwendung nach Anspruch 12, oder nicht therapeutische Verwendung einer Zusammensetzung nach Anspruch 12, wobei das Hyaluronsäure:Dermatansulphat-Gewichtsverhältnis 1:0,25 ist.

14. Zusammensetzung für deren Verwendung nach einem der Ansprüche 1 bis 7, Anspruch 12 oder Anspruch 13, oder nicht therapeutische Verwendung einer Zusammensetzung nach einem der Ansprüche 8 bis 13, zusätzlich umfassend Kollagenhydrolysat.

15. Zusammensetzung für deren Verwendung oder nicht therapeutische Verwendung einer Zusammensetzung nach Anspruch 14, wobei die Hyaluronsäure, das Dermatansulphat und das Kollagenhydrolysat bei einem Hyaluronsäure:Dermatansulphat:Kollagenhydrolysat-Gewichtsverhältnis vorhanden sind, welches zwischen 1:0,06:0,03 und 1:0,80:0,40, vorzugsweise zwischen 1:0,10:0,05 und 1:0,50:0,20, liegt.

16. Zusammensetzung für deren Verwendung oder nicht therapeutische Verwendung einer Zusammensetzung nach Anspruch 15, wobei das Hyaluronsäure:Dermatansulphat:Kollagenhydrolysat-Gewichtsverhältnis 1:0,20:0,10 ist.

## Revendications

1. Composition comprenant de l'acide hyaluronique et du dermatane sulfate destinée à être utilisée dans le traitement ou la prévention de l'obésité, la résistance à l'insuline, le diabète de type 2, la stéatose hépatique ou la dyslipidémie chez un mammifère.

2. Composition destiné à être utilisée selon la revendication 1, dans laquelle la résistance à l'insuline est associée au surpoids ou à l'obésité, le diabète de type 2 est associé au surpoids ou à l'obésité, la stéatose hépatique est associé au surpoids ou à l'obésité ou la dyslipidémie est associée au surpoids ou à l'obésité.

3. Composition destinée à être utilisée selon la revendication 1 ou la revendication 2, dans laquelle la dyslipidémie est de l'hypercholestérolémie et/ou de l'hypertriglycéridémie.

4. Composition destinée à être utilisée selon la revendication 1 ou la revendication 2, dans laquelle le traitement ou la prévention de la dyslipidémie occasionne la réduction du cholestérol sanguin, le maintient de niveaux normaux de cholestérol sanguin, la réduction des triglycérides sanguins ou le maintient de niveaux normaux de triglycérides sanguins.

5. Composition destinée à être utilisée selon la revendication 1 ou la revendication 2, dans laquelle le traitement ou la prévention de la résistance à l'insuline ou du diabète de type 2 occasionne une augmentation de la sensibilité à l'insuline.

6. Composition destinée à être utilisée selon l'une quelconque des revendications 1 à 5, dans laquelle la composition se présente sous forme d'une composition pharmaceutique ou un aliment médical.

7. Composition destinée à être utilisée selon l'une quelconque des revendications 1 à 6, dans laquelle le mammifère est obèse ou il a du surpoids.

8. Utilisation non thérapeutique d'une composition comprenant de l'acide hyaluronique et du dermatane sulfate comme aliment, aliment fonctionnel ou supplément alimentaire, pour la réduction du surpoids, la réduction de l'apport alimentaire, l'induction de la sensation de satiété, la réduction de l'appétit, la réduction du poids corporel, la prévention de la prise de poids, la réduction de la graisse corporelle ou la réduction de la formation de graisse chez un mammifère.

9. Utilisation non thérapeutique d'une composition selon la revendication 8, dans laquelle le mammifère a du surpoids.

10. Utilisation non thérapeutique d'une composition comprenant de l'acide hyaluronique et du dermatane sulfate comme produit amincissant.

11. Utilisation non thérapeutique d'une composition selon la revendication 10, dans laquelle la composition se trouve sous forme d'un aliment, un aliment fonctionnel ou un supplément alimentaire.

12. Composition destinée à être utilisée selon l'une quelconque des revendications 1 à 7, ou utilisation non thérapeutique d'une composition selon l'une quelconque des revendications 8 à 11, dans laquelle l'acide hyaluronique et le dermatane sulfate sont présents dans un rapport en poids d'acide hyaluronique : dermatane sulfate compris entre 1 : 0,06 et 1 : 0,80, de préférence entre 1 : 0,10 et 1 : 0,50.

13. Composition destinée à être utilisée selon la revendication 12, ou utilisation non thérapeutique d'une composition selon la revendication 12, dans laquelle le rapport en poids d'acide hyaluronique : dermatane sulfate est 1 : 0,25.

14. Composition destinée à être utilisée selon l'une quelconque des revendications 1 à 7, la revendication 12 ou la revendication 13, ou utilisation non thérapeutique d'une composition selon l'une quelconque des revendications 8 à 13, comprenant en outre de l'hydrolysat de collagène.

15. Composition destinée à être utilisée ou utilisation non thérapeutique d'une composition selon la revendication 14, dans laquelle l'acide hyaluronique, le dermatane sulfate et l'hydrolysat de collagène sont présents dans un rapport en poids d'acide hyaluronique : dermatane sulfate : hydrolysat de collagène compris entre 1 : 0,06 : 0,03 et 1 : 0,80 : 0,40, de préférence entre 1 : 0,10 : 0,05 et 1 : 0,50 : 0,20.

16. Composition destinée à être utilisée ou utilisation non thérapeutique d'une composition selon la revendication 15, dans laquelle le rapport en poids d'acide hyaluronique : dermatane sulfate : hydrolysat de collagène est 1 : 0,20 : 0,10.
